# EUROPEAN PATENT SPECIFICATION

(11) **EP 0 604 354 B1**
(45) Date of publication and mention of the grant of the patent: **15.01.2003**
(21) Application number: 93610065.0
(22) Date of filing: 20.12.1993
(51) Int. Cl.: C07D 451/02, A61K 31/46

(54) **Antidepressant and antiparkinsonian compounds**
Antidepressiv und gegen die Parkinsonsche Krankheit wirkende Verbindungen
Composés antidépressifs et anti-parkinsoniens

(30) Priority: 23.12.1992 DK 154192; 16.07.1993 US 93182
(43) Date of publication of application: 29.06.1994
(73) Proprietor: NEUROSEARCH A/S, 2750 Ballerup (DK)
(72) Inventor: Moldt, Peter, DK-3050 Humlebaek (DK); Scheel-Krüger, Jorgen, DK-2600 Glostrup (DK); Jensen, Leif Helth, DK-1573 Copenhagen V (DK)

(56) References cited:
- EP-A- 0 239 309
- EP-A- 0 316 718
- EP-A- 0 327 155
- EP-A- 0 398 578
- WO-A-92/03433
- WO-A-93/09814
- WO-A-93/18033
- DE-B- 2 530 005
- US-A- 3 813 404
- US-A- 3 901 892
- US-A- 5 268 480
- JOURNAL OF MEDICINAL CHEMISTRY, vol.36, no.7, 1993 pages 855 - 862 P.C. MELTZER ET AL. 'Substituted 3-Phenyltropane Analogs...'
- JOURNAL OF THE CHEMICAL SOCIETY, CHEMICAL COMMUNICATIONS, no.1, January 1993 pages 44 - 46 F.I. CARROLL ET AL. 'Synthesis and Cocaine Receptor Affinities...'
- JOURNAL OF MEDICINAL CHEMISTRY, vol.16, no.11, 1973 pages 1260 - 1267 R.L. CLARKE ET AL. 'Compounds Affecting the Central Nervous System...'
- JOURNAL OF MEDICINAL CHEMISTRY, vol.34, no.3, 1991 pages 883 - 886 F.I. CARROLL ET AL. 'Synthesis and Ligand Binding...'
- J. Med. Chem. 1992, 35, 969 - 981

## Description

The present invention relates to: novel 2,3-trans-isomeric tropane derivatives which have pronounced anti drug abuse, antidepressant and anti-Parkinsonian activity and, at the same time, a low degree of undesired side effects; methods for the preparation of the novel tropane derivatives; pharmaceutical compositions containing the novel tropane derivatives.

### Objects of the Invention

It is an object of the present invention to provide novel tropane derivatives having anti drug abuse, antidepressant and anti-Parkinsonian activity.

Another object of the invention is to provide novel pharmaceutical compositions containing the novel tropane derivatives which are useful for the treatment of drug abuse, depression and Parkinsonism.

Other objects will become apparent hereinafter to one skilled in the art.

### Background of the Invention

It is well known, that cocaine has a varity of pharmacological actions, primarily a strong CNS stimulation and local anesthetic action. These effects are accompanied by high toxicity and dependence liability. (see for example R.L.Clarke et al in Journal of Medicinal Chemistry 16(11), 1261-1267 (1973). The dependence liability is thought to be related to a combination of cocaine's powerful stimulant activity, short term of action, and rapid onset of action together with its strong dopamine-releasing properties. Further it is well known, that cocaine also possess powerful dopamine reuptake inhibiting activity. It is believed, that compounds having long lasting selective dopamine reuptake-inhibiting properties, and being devoid of dopamine releasing properties, will be extremely useful as a novel type of antidepressant and anti-Parkinsonian agent. Furthermore such compounds will be extremely useful in the treatment of drug addiction, and especially in the treatment of cocaine addiction or misuse.

During the years many attempts have been made to optimize upon the properties of cocaine. Many derivatives of cocaine and of its isomers have been synthesized. See for example R.L.Clarke et al in Journal of Medicinal Chemistry 16(11), 1261-1267 (1973) and F. Ivy Caroll et al in Journal of Medicinal Chemistry 34, 883-886 (1991). Also F.lvy Carroll et al in Journal of Medicinal Chemistry 35 (6) 969-981 (1992) describes cocaine analogues that act as dopamine reuptake inhibitors. Many of these derivatives and probably most pronounced the derivatives of R.L.Clarke et al above are very powerful stimulant compounds and have been found to be very potent dopamine reuptake inhibitors. However none of the cocaine derivatives synthesized until today have been found to be devoid of undesired side effects. R.L.Clarke et al in above paper notes at page 1265, that an enantiomer (trans isomer) of a closely related compound (cis isomer as is cocaine) was not stimulant, and at the same page, that moving a 2-carboxy function from an axial (cis isomer) to an equatorial configuration (trans isomer) gave a compound which was inactive in the locomotor screen (>256mg/kg), but appeared to produce a slight stimulation. P.C. Meltzer et al in Journal og Medicinal Chemistry, 36(7), 855-862 (1993) notes that the trans isomers are biologically inactive.

Throughout the literature all efforts of derivating cocaine have focused upon optimizing the dopamine reuptake inhibiting properties and ligand affinity by synthesizing further cis isomers (just as cocaine), undoubtedly because these compounds have been found to be the most potent dopamine reuptake inhibitors as well as the most potent ligands in various binding assays. This also includes the findings published by F. Ivy Carroll et al in J. Chem. Soc. Chem. Commun. pp. 44-46 (1993).

### The present Invention

The inventors to the present invention became scientifically interested in the compounds of R.L.Clarke et al and decided to synthesize the compound designated No. 13 by Clarke et al (Win 35,428). During the syntheses of Win 35,428, the trans isomer of Win 35,428 was isolated as a side product by the present inventors. During a careful characterization of both of these epimeric compounds it was surprisingly noted, that the trans isomer, though being only close to 50 times less potent as compared to the cis isomer (Win 35,428) as a dopamine reuptake inhibitor, then in contrast to Win 35,428, at every relevant dosing level tested appeared essentially or totally devoid of side effects as measured by excitatory locomotor activity and rearing responses as well as by abnormal stereotyped behaviour, and as well as the fact that the compound was essentially free of dopamine releasing properties, all in strong contrast to Win 35,428 and cocaine.

These new and surprising findings prompted the inventors of the present invention to synthesize a large series of derivatives of Win 35,428, its isomers and furthermore more remotely related compounds.

It was thereby surprisingly found that, by selecting the trans isomer, rather than the cis isomer as appearing throughout the literature, and by substituting the CO₂Me group of Win 35,428 for various other substituents and most pronounced for various sterically large substituents, it became possible at one and the same time, to maintain and enhance the dopamine reuptake inhibiting properties of the compounds, as well as to further reduce or eliminate the side effects in the form of excitatory locomotor activity and rearing responses as well as by way of abnormal stereotyped behaviour. Further it was found that the compounds were devoid of dopamine releasing properties. Most surprisingly a strong and potent anti-Parkinsonian and antidepresant activity remained with the compounds. Certain of the compounds provided herewith also possess very potent serotonine (5-hydroxythryptamine, 5-HT) reuptake inhibiting activity in combination with their potent dopamine reuptake inhibiting activity. While it is well established that either selective dopamine reuptake inhibitors or selective serotonine reuptake inhibitors are efficient antidepressive agents, then it is very often necessary to use unselective dopamine/serotonine uptake inhibitory compounds, such as imipramine and amitriptyline, to stabilize mood in severely depressed patients. Therefore compounds of the invention exhibiting this combination of activity may also be effective in mood stabilization of such severely depressed patients. An example of such a compound is (1R,2R,3S)-2-(4-Chlorophenoxy-methyl)-3-(4-fluorophenyl)tropane.

The invention then, inter alia, comprises the following, alone or in combination:

A 2,3-trans-isomeric tropane derivative having the formula 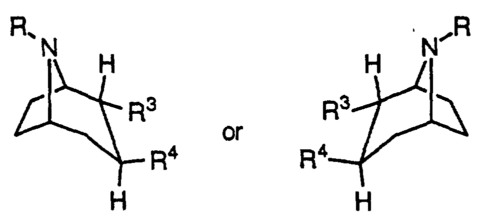 any mixture thereof, or a pharmaceutically acceptable salt thereof; wherein
R is hydrogen or C₁₋₆-alkyl;
R³ is CH₂-O-phenyl or CH₂-O-CO-phenyl, which phenyl groups may be substituted one or more times with substituents selected from the group consisting of halogen, CF₃, CN, C₁₋₆-alkoxy, C₁₋₆-alkyl, NH₂, or nitro; and
R⁴ is phenyl or benzyl, which phenyl or benzyl groups may be substituted one or more times with substituents selected from the group consisting of halogen, CF₃, CN, C₁₋₆-alkoxy, C₁₋₆-alkyl, NH₂, or nitro, and
a compound which is (1R,2R,3S)-2-(4-Chlorophenoxy-methyl)-3-(4-fluorophenyl)tropane; or a pharmaceutically-acceptable addition salt thereof, and
the use of a compound of the invention, for the manufacture of a medicament for the treatment of a disorder or disease of a living animal body, including a human, which disorder or disease is responsive to the inhibition of dopamine reuptake in the central nervous system, and
the use of a compound of the invention, for the manufacture of a medicament for the treatment of depression or Parkinsonism, and
the use of a compound of the invention, for the manufacture of a medicament for the treatment of drug addiction and/or abuse,
and the use of a compound of the invention, for the manufacture of a medicament for the treatment of cocaine and/or amphetamine addiction and/or abuse, and
a pharmaceutical composition, comprising an effective amount of a compound as any above, or a pharmaceutically-acceptable addition salt thereof, together with at least one pharmaceutically-acceptable carrier or diluent, and
a metod of preparing a compound having the formula 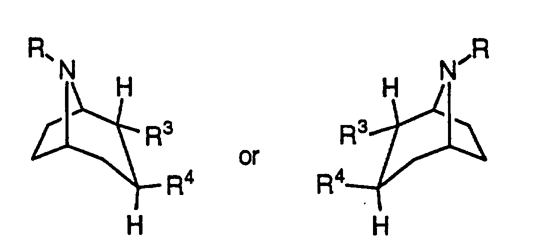 any mixture thereof, or a pharmaceutically acceptable salt thereof; wherein
R,
R³, and
R⁴ are as defined in claim 1 comprising the step of reacting acompound having the formula 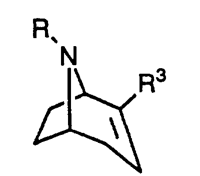 wherein R and R³ have the meanings set forth above, with a compound having the formula R⁴-A, wherein R⁴ has the meaning set forth above and wherein A is any type of reactive functionality suitable for generating a carbanion as its counterpart, such as Li, MgX, wherein X is halogen, and CuLi, in a Michael like 1,4 addition reaction, to form a compound of the invention in a one step procedure, and
the method of above wherein (1R,2R,3S)-2-(4-Chlorophenoxy-methyl)-3-(4-fluorophenyl)tropane; or a pharmaceutically-acceptable addition salt thereof, is prepared.

Examples of pharmaceutically-acceptable addition salts include inorganic and organic acid addition salts such as the hydrochloride, hydrobromide, phosphate, nitrate, perchlorate, sulphate, citrate, lactate, tartrate, maleate, fumarate, mandelate, benzoate, ascorbate, cinnamate, benzenesulfonate, methanesulfonate, stearate, succinate, glutamate, glycollate, toluene-p-sulphonate, formate, malonate, naphthalene-2-sulphonate, salicylate and the acetate. Such salts are formed by procedures well known in the art.

Other acids such as oxalic acid, while not in themselves pharmaceutically acceptable may be useful in the preparation of salts useful as intermediates in obtaining compounds of the invention and their pharmaceutically acceptable acid addition salts.

Halogen is fluorine, chlorine, bromine, or iodine; chlorine and bromine are preferred.

Alkyl means a straight chain or branched chain of one to six carbon atoms or cyclic alkyl of from three to seven carbon atoms, including methyl, ethyl, propyl, isopropyl, butyl, isobutyl, t-butyl, pentyl, hexyl, cyclopropyl, cyclobutyl, cyclopentyl, cyclohexyl; methyl, ethyl, propyl and isopropyl are preferred groups.
I.p. means intraperetoneally, which is a well known route of administration.
P.o. means peroral, which is a well known route of administration.

Further, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

It will be appreciated by those skilled in the art that some of the compounds of the present invention contain at least one chiral centre and that such compounds exist in the form of a pair of optical isomers (i.e. enantiomers). The invention includes all such isomers and mixtures thereof including racemic mixtures.

Some of the compounds of the present invention exist in (+) and (-) forms as well as in racemic forms. Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can thus be resolved into their optical antipodes, e.g., by fractional crystallization of d- or I- (tartrates, mandelates, or camphorsulphonate) salts for example. The compounds of the present invention may also be resolved by the formation of diastereomeric amides by reaction of the compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the compounds of the present invention with an optically active chloroformate or the like.

Additional methods for the resolvation of optical isomers, known to those skilled in the art may be used, and will be apparent to the average worker skilled in the art. Such methods include those discussed by J. Jaques, A. Collet, and S. Wilen in "Enantiomers, Racemates, and Resolutions", John Wiley and Sons, New York (1981).

A method of preparing the compounds of the invention comprises the step of reacting a compound having the formula 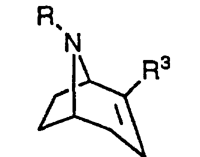 wherein R and R³ have the meanings set forth above, with a compound having the formula R⁴-A, wherein R⁴ has the meaning set forth above and wherein A is any type of reactive functionality suitable for generating a carbanion as its counterpart, such as Li, MgX, wherein X is halogen, and CuLi, in a Michael like 1,4 addition reaction, to form a compound of the invention in a one step procedure.

Starting materials for the processes described in the present application are known or can be prepared by known processes from commercially available chemicals.

The products of the reactions described herein are isolated by conventional means such as extraction, crystallization, distillation, chromatography, and the like.

The compounds of the invention and their pharmaceutically acceptable derivatives may be prepared by any method known in the art for the preparation of compounds of the invention and compounds of analogous structure, as shown in the representative examples which follow.

### Biology

The compounds of the present invention are potent dopamine reuptake inhibitors.

The compounds of the present invention have been tested for their ability to inhibit reuptake of dopamine in synaptosomes of rat forebrain.

### Test procedure

Fresh whole forebrain (at 0-4°C) from male Wistar rats (150-200 g) is homogenized in a glass homogenizer with a teflon pestle in 20 x volumes 0.32M sucrose. The homogenate is centrifuged at 1000 x g (∼ 2900 rpm) for 10 min. The pellet is discarded and the supernatant is used for uptake assays.

A Krebs Buffer (119 mM NaCI, 24 mM NaHCO₃, 2.1 mM KCI, 1.2 mM KH₂PO₄, 1.8 mM CaCl₂, 2 mM MgSO₄, 7H₂O, 12 mM glucose) is equilibrated with an atmosphere of 96% O₂: 4% CO₂ for at least 60 min at 37°C. To 5 ml Krebs-buffer 100 µl of the radioactivity is added (3H-dopamine 1 x 10⁻⁹M final concentration) and 100 µl test substance solution and 200 µl of tissue suspension. The samples are thoroughly mixed and incubated 10 min at 37°C followed by 2 min at 0°C. After incubation the samples are poured directly onto Whatman GF/C glass fibre filters under suction and immediately washed with 2 x 5 ml ice-cold 0.9% saline (NaCI) buffer. To the filter is added 3 ml of scintillation fluid, and the amount of radioactivity on the filters is determined by conventional liquid scintillation counting. The test value is given as the IC₅₀ (the concentration (µM) of the test substance which inhibits the uptake by 50%). Test results obtained by testing selected compounds of the present invention will appear from the below table:

**Table**

| Compound | Dopamine reuptake inhibiting activity |
|---|---|
| (1R,2R,3S)-2-(4-Chlorophenoxymethyl)-3-(4-fluorophenyl)tropane | 0.39µM |

The compounds of the present invention have been tested for their antiparkinsonian activity by their ability to antagonize haloperidol-induced catalepsy. This model is recognized in the art as a very reliable model for antiparkinsonian activity.

### The Catalepsy test for antiparkinsonian effects.

The principle behind the haloperidol catalepsy test relates to the fact that the drug haloperidol (Serenase®) induces a receptor blockade of post synaptic dopamine receptors within the dopamine innervated corpus striatum of the rat brain. Following the blockade of active dopamine neurotransmission of the striatum a state of rigid immobility is induced, during which the rat can be placed in various totally abnormal immobile postures involving the forelegs and the whole body. These immobile postures can also be produced by high doses of haloperidol to several other animal species(mice, dog) as well as primates, including humans. The immobile posture effect induced by haloperidol closely resembles the state of Parkinson's disease in humans, which is due to a deficit of the dopaminergic innervation of the striatal complex (nucleus caudatus, putamen).

The day before testing, the rats weighing 200-250 g are housed 2 and 2 together in standard macrolon cages.
The test substance is usually given perorally 1 h before a standard dose of the dopamine antagonist haloperidol 0.25 mg/kg given subcutaneously.
The testing for the immobile catalepsy syndrome includes 4 tests performed in the following consecutive order:
1) a vertical wire netting (40x40 cm high).The meshes (openings) of the nettings are approximately 1x2cm.
2) a horizontal bar 9 cm above the floor
3) a 9 cm high block (bar)
4) a 3 cm high block (cork)

The rats were scored for the immobile cataleptic syndrome every 15 min in all 4 tests starting 15 min after the haloperidol injection.
The intensity of catalepsy was tested during 10 sec in all tests and evaluated according to a criteria of 10 sec of total immobility for a score of 2. Minor movements of the head or the body give the score of 1 and a score of 0 is given if the rat shows no symptoms.

The rat was placed in the middle of the vertical wire netting, then on the horizontal bar in an extended position with support by both the forelegs on the bar.
The rats were then tested, whether or not they were willing to sit with the left or right forelegs placed first on the 9 and then on the 3 cm block for a duration of 10 sec. The maximum score for all 4 tests is thus a total of 8.

**Table**

| Compound | Minimum effective dose (peroral administration) |
|---|---|
| (1R,2R,3S)-2-(4-Chlorophenoxymethyl)-3-(4-fluorophenyl)tropane | < 1 mg/kg |

### Side effect profile of the compounds of the present invention

The side effects of cocaine and the central stimulant amphetamine derivatives involve central excitement and stimulation in animals including primates and these effects can also be observed in humans. A serious side effect of cocaine and of the amphetamine derivatives includes also the ability to provoke toxic psychotic symptoms closely resembling the mental disease schizophrenia and these include halucinations, paranoia and abnormal bizarre stereotyped mental activity and stereotyped motor activity.

The current knowledge strongly indicates and suggests that these syndromes in primates and in humans are due to an extensive and massive release of dopamine within the striatal complex and in particular within the mesolimbic dopamine system, which innervate limbic structures including the nucleus accumbens.

The induction of stereotyped abnormal behavior in rodents thus also represents one of the most used animal models of schizophrenia in models for antipsychotic neuroleptic drugs (including haloperidol and chlorpromazine).

The development of toxic abnormal stereotyped amphetamine syndrome as described below may predict a toxic central stimulant side effect of dopamine releasing compounds in humans.

### Classification of the behavioural excitatory locomotor and rearing responses after amphetamine, cocaine and various dopamine uptake inhibitors.

Male SPF wistar rats weighing 200-250g were used for the gross behavioural studies. All rats were transferred the day before the experiments to individual cages made of wire netting (floor area 21 x 27 cm, height 16 cm) at a room temperature of 21-23° C. As a rule the rats were observed continuously for 5-6 hrs after the administration of the drugs. The behavioural elements here classified as rearing (i.e. standing up on the hindlegs) and locomotor activities (i.e. forward running activities) were registered, and the maximum peak effects were classified according to the following rating scale:
0 = non-existent or very infrequent
+ = weak and infrequent
++ = moderate in intensity and frequency
+++ = very strong, continuous and intense

The degree of locomotor activity was also tested and measured quantitatively in photocell activity cages. For the measurement of this locomotor activity 100 g female rats are used.

**Table**

| Compound | Dose(i.p.) | Activity |
|---|---|---|
| (1R,2R,3S)-2-(4-Chlorophenoxymethyl)-3-(4-fluorophenyl)tropane | 50 mg/kg | 0 |

### Classification of the abnormal stereotyped behaviour.

In general, the abnormal stereotyped behaviour after administration of amphetamine and cocaine-like central stimulant drugs can be classified into "low" and "high" intensity scores of stereotyped behaviour. The low intensity score of stereotypy includes an abnormal and continuous repetition of the locomotor, rearing and sniffing behaviour, and these syndromes are usually seen only after the lower doses of the central dopaminergic central stimulant drugs or may be seen present during the pre- and afterphases of the high doses. The low intensity behavioural effects are here included into the locomotor and rearing syndrome. The high intensity syndrome of stereotypy is here considered, if the behavioural repertoire of the rat becomes strongly restricted in variation and consists of the continuous repetition of one or a few items of behaviour.
The syndrome of stereotyped sniffing behaviour is thus performed continuously on only a small restricted area of the cage. This activity usually starts on the upper part of the wall and following higher doses of the drugs increases in intensity to the performance of sniffing towards the lower part of the cage on the wall or on the wires of the floor. During this stage of high intensity stereotypy all normal behavioural elements are absent including behaviour such as eating, drinking, grooming and normal explorative investigation of the environment. In rats, the high intensity sniffing can develop into sniffing associated with licking and/or biting-gnawing activity on the wire netting of the cage following still higher doses of the stimulant drugs. The rats are here usually sitting in a typical crouched posture in a comer of the cage. Backward locomotion may occasionally be observed.

The following rating scale is used for the high intensity stereotypy on the condition that the behavioural syndromes are as decribed above:
+ = only stereotyped sniffing
++ = stereotyped sniffing and episodic licking
+++ = Continuous licking and/or biting gnawing

**Table**

| Compound | Dose(i.p.) | Activity |
|---|---|---|
| (1R,2R,3S)-2-(4-Chlorophenoxymethyl)-3-(4-fluorophenyl)tropane | 50 mg/kg | 0 |

### Pharmaceutical Compositions

While it is possible that, for use in therapy, a compound of the invention may be administered as the raw chemical, it is preferable to present the active ingredient as a pharmaceutical formulation.

The invention thus further provides pharmaceutical formulations comprising a compound of the invention or a pharmaceutically acceptable salt or derivative thereof together with one or more pharmaceutically acceptable carriers therefor and, optionally, other therapeutic and/or prophylactic ingredients. The carrier(s) must be "acceptable" in the sense of being compatible with the other ingredients of the formulation and not deleterious to the recipient thereof.

Pharmaceutical formulations include those suitable for oral, rectal, nasal, topical (including buccal and sub-lingual), vaginal or parenteral (including intramuscular, sub-cutaneous and intravenous) administration or in a form suitable for administration by inhalation or insufflation.

The compounds of the invention, together with a conventional adjuvant, carrier, or diluent, may thus be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Formulations containing ten (10) milligrams of active ingredient or, more broadly, 0.1 to one hundred (100) milligrams, per tablet, are accordingly suitable representative unit dosage forms.

The compounds of the present invention can be administrated in a wide variety of oral and parenteral dosage forms. It will be obvious to those skilled in the art that the following dosage forms may comprise, as the active component, either a compound of the invention or a pharmaceutically acceptable salt of a compound of the invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in admixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration may be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution.

The compounds according to the present invention may thus be formulated for parenteral administration (e.g. by injection, for example bolus injection or continuous infusion) and may be presented in unit dose form in ampoules, prefilled syringes, small volume infusion or in multi-dose containers with an added preservative. The compositions may take such forms as suspensions, solutions, or emulsions in oily or aqueous vehicles, and may contain formulatory agents such as suspending, stabilising and/or dispersing agents. Alternatively, the active ingredient may be in powder form, obtained by aseptic isolation of sterile solid or by lyophilisation from solution, for constitution with a suitable vehicle, e.g. sterile, pyrogen-free water, before use.

Aqueous solutions suitable for oral use can be prepared by dissolving the active component in water and adding suitable colorants, flavours, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilizers, buffers, artificial and natural sweeteners, dispersants, thickeners, solubilizing agents, and the like.

For topical administration to the epidermis the compounds according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Formulations suitable for topical administration in the mouth include lozenges comprising active agent in a flavoured base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations may be provided in single or multidose form. In the latter case of a dropper or pipette, this may be achieved by the patient administering an appropriate, predetermined volume of the solution or suspension. In the case of a spray this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In formulations intended for administration to the respiratory tract, including intranasal formulations, the compound will generally have a small particle size for example of the order of 5 microns or less. Such a particle size may be obtained by means known in the art, for example by micronization.

When desired, formulations adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The following examples will illustrate the invention further .

### Example 1

### (Synthesis of intermediate compound)

### (-)-Anhydroecgonine methyl ester.

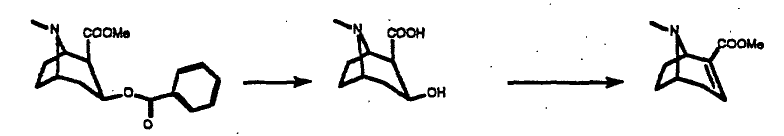

(1R,2R,3S)-2-Carbomethoxy-3-benzoxytropane, hydrochloride (100 g, 0.29 mol) was refluxed in 1000 ml 1 M hydrochloric acid for 18 hours and the solution was ice cooled. Benzoic acid was collected by filtration and the filtrate was concentrated in vacuo. Trituration of the remanescens with ethanol and filtration yields (1R,2R,3S)-3-hydroxy-tropane 2 carboxylate, hydrochloride as a white crystalline compound which without further purification was dried and refluxed in phosphorous oxychloride (50 ml) for two hours. The solution was concentrated in vacuo and absolute methanol (150 ml) was slowly added under ice cooling. The solution was stirred at ambient temperature for 16 hours and was concentrated in vacuo. The remanescens was ice cooled and made basic by addition of a sodium hydroxide solution (10 M, approximately 100 ml) and was extracted 5 times with diethyl ether. The combined organic phase was dried and concentrated in vacuo yielding an oil, which was distilled in vacuo (70-74° C, 1 mBar) yielding the title compound as a clear oil.

### Example 2

### (Synthesis of intermediate compounds)

(1R,2R,3S)-2-Hydroxymethyl-3-(4-fluorophenyl)tropane, white crystals, m.p. 169-170° C, was made in a similar way to the following compound: (1R,2S,3S)-2-Hydroxymethyl-3-(4-fluorophenyl)tropane.

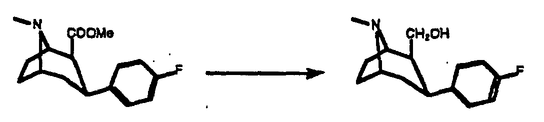

To a suspension of lithium aluminum hydride (0.8 g, 21 mmol) in diethyl ether (30 ml), at room temperature, was slowly added a solution of (1R,2S,3S)-2-carbomethoxy-3-(4-fluorophenyl)tropane (5 g, 18 mmol) in 100 ml diethyl ether. The reaction completes after stirring for 10 minutes and was quenched by addition of 0.8 ml water, 0.8 ml sodium hydroxide (15%) and 2 ml water. The aluminum salts were removed by filtration and the solvent was removed in vacuo leaving an oil. The title compound precipitates upon trituration with pentane as white crystals, m.p. 79-80° C.

### Example 3

(1R,2R,3S)-2-(4-Chlorophenoxy-methyl)-3-(4-fluorophenyl)tropane, hydrochloride.

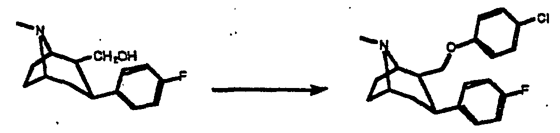

A mixture of (1R,2R,3S)-2-hydroxymethyl-3-(4-fluorophenyl)tropane (1.8 g, 7.2 mmol), *p*-toluen-sulphonylchloride (2 g, 10.5 mmol) and triethyl amine (1.6 ml, 12 mmol) in methylene chloride (50 ml) was stirred at room temperature for 20 hours and the reaction mixture was concentrated in vacuo. Water (50 ml) and diethyl ether (50 ml) was added and the mixture was stirred for 1/2 hour. The organic phase was washed once with sodium hydroxide (1M), twice with water, and was then dried and concentrated in vacuo. The remanescens was dissolved in a small amount of absolute ethanol and was, at room temperature, added to a solution of sodium 4-chloro-phenolate in absolute ethanol, made by addition of sodium (0.24 g, 10 mmol) to a solution of *p-*chlorophenol in absolute ethanol. The mixture was refluxed for three days and was concentrated in vacuo. The remanescens was stirred in a mixture of water and diethyl ether and the organic phase was washed with water. After drying and concentration in vacuo, the crude product was subjected to column chromatography first with ethyl acetate and later with ethyl acetate and methanol (10%) as eluent. An oil was isolated which was dissolved in a small amount of diethyl ether and the title compound was precipitated, by addition of hydrochloric acid in diethyl ether, as white crystals (hygroscopic), m.p.
70-75°C.

The following compounds were made in a similar way:
(1R,2R,3S)-2-(4-Chlorophenoxy-methyl)-3-(3,4-dichlorophenyl)tropane, hydrochloride, white crystals (hygroscopic), m.p. 45-50° C.
(1R,2R,3S)-2-(4-Chlorophenoxy-methyl)-3-(4-methylphenyl)tropane, hydrochloride, white crystals (hygroscopic), m.p. 65-70° C.

### Example 4

(1R,2R,3S)-2-(Benzoyloxy-methyl)-3-(4-fluorophenyl)tropane, hydrochloride, white crystals, m.p. 90-91°C, was made in a similar way to the following compound: (1R,2S,3S)-2-(Benzoyloxy-methyl)-3-(4-fluorophenyl)tropane, hydrochloride.

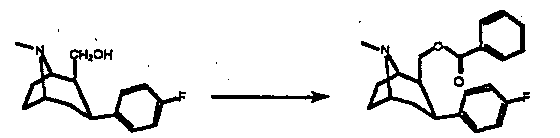

To a mixture of (1R,2S,3S)-2-hydroxymethyl-3-(4-fluorophenyl)tropane (0.5 g, 2 mmol) and potassium carbonate (0.35 g, 2.5 mmol) in tetrahydrofurane (25 ml), at room temperature, was slowly added a solution of benzoyl chloride (0.3 ml, 2.5 mmol) in tetrahydrofurane (5 ml). The reaction mixture was stirred for to hours at ambient temperature and was concentrated in vacuo. Water (20 ml) was added and the suspension was extracted twice with diethyl ether. The organic phase was dried and was concentrated in vacuo. Thereafter the crude product was dissolved in a small amount of diethyl ether and the title compound was precipitated, by addition of hydrochloric acid in diethyl ether, as white crystals, m.p. 230-233°C.

## Claims

1. A 2,3-*trans*-isomeric tropane derivative having the formula 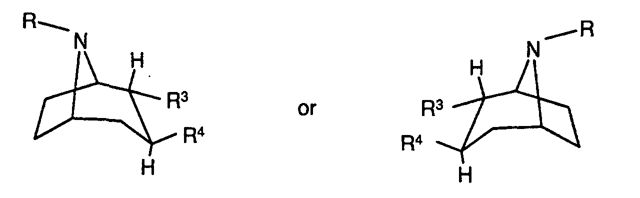 any mixture thereof, or a pharmaceutically acceptable salt thereof; wherein
R is hydrogen or C₁₋₆-alkyl;
R³ is CH₂-O-phenyl or CH₂-O-CO-phenyl, which phenyl groups may be substituted one or more times with substituents selected from the group consisting of halogen, CF₃, CN, C₁₋₆-alkoxy, C₁₋₆-alkyl, NH₂, or nitro; and
R⁴ is phenyl or benzyl, which phenyl or benzyl groups may be substituted one or more times with substituents selected from the group consisting of halogen, CF₃, CN, C₁₋₆-alkoxy, C₁₋₆-alkyl, NH₂, or nitro.

2. The tropane derivative of claim 1, which is
(1R,2R,3S)-2-(4-Chlorophenoxy-methyl)-3-(4-fluorophenyl)tropane;
(1R,2R,3S)-2-(4-Chlorophenoxy-methyl)-3-(3,4-dichlorophenyl)tropane;
(1R,2R,3S)-2-(4-Chlorophenoxy-methyl)-3-(4-methylphenyl)tropane; or
(1R,2R,3S)-2-(Benzoyloxy-methyl)-3-(4-fluorophenyl)tropane;
or a pharmaceutically-acceptable addition salt thereof.

3. The use of a tropane derivative according to any of claims 1-2, for the manufacture of a medicament for the treatment of a disorder or disease of a living animal body, including a human, which disorder or disease is responsive to the inhibition of dopamine reuptake in the central nervous system.

4. The use of a tropane derivative according to any of claims 1-2, for the manufacture of a medicament for the treatment of depression or Parkinsonism.

5. The use of a tropane derivative according to any of claims 1-2, for the manufacture of a medicament for the treatment of drug addiction and/or abuse.

6. The use of a tropane derivative according to any of claims 1-2, for the manufacture of a medicament for the treatment of cocaine and/or amphetamine addiction and/or abuse.

7. The use according to any of claims 3-6, wherein the tropane derivative employed is
(1R,2R,3S)-2-(4-Chlorophenoxy-methyl)-3-(4-fluorophenyl)tropane;
(1R,2R,3S)-2-(4-Chlorophenoxy-methyl)-3-(3,4-dichlorophenyl)tropane;
(1R,2R,3S)-2-(4-Chlorophenoxy-methyl)-3-(4-methylphenyl)tropane; or
(1R,2R,3S)-2-(Benzoyloxy-methyl)-3-(4-fluorophenyl)tropane;
or a pharmaceutically-acceptable addition salt thereof.

8. A pharmaceutical composition, comprising an effective amount of a tropane derivative of any of claims 1-2, or a pharmaceutically-acceptable addition salt thereof, together with at least one pharmaceutically-acceptable carrier or diluent.

9. A method of preparing a tropane derivative of any of claims 1-2, which method comprises the step of reacting a compound having the formula 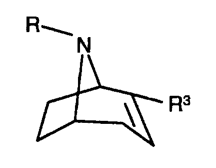 wherein R and R³ have the meanings set forth in claim 1,
with a compound having the formula R⁴-A,
wherein R⁴ has the meaning set forth in claim 1, and wherein A is any type of reactive functionality suitable for generating a carbanion as its counterpart, such as Li, MgX,
wherein X is halogen, and CuLi, in a Michael like 1,4 addition reaction,
to form a tropane derivative of the invention in a one step procedure.

## Patentansprüche

1. Ein 2,3-*trans*-isomeres Tropanderivat mit der folgenden Formel 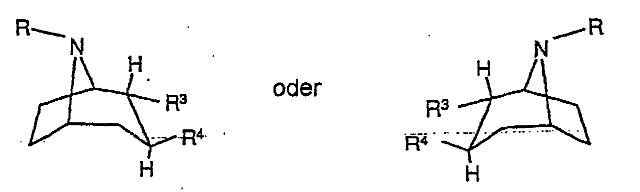 eine beliebige Mischung davon oder ein pharmazeutisch verträgliches Salz davon; worin
R Wasserstoff oder C₁₋₆-Alkyl darstellt;
R³ CH₂-O-Phenyl oder CH₂-O-CO-Phenyl darstellt, wobei die Phenylgruppen einmal oder mehrmals mit Substituenten, ausgewählt aus der Gruppe, bestehend aus Halogen, CF₃, CN, C₁₋₆-Alkoxy, C₁₋₆-Alkyl, NH₂ oder Nitro, substituiert sein können; und
R⁴ Phenyl oder Benzyl darstellt, wobei die Phenyl- oder Benzylgruppen einmal oder mehrmals mit Substituenten, ausgewählt aus der Gruppe, bestehend aus Halogen, CF₃, CN, C₁₋₆-Alkoxy, C₁₋₆-Alkyl, NH₂ oder Nitro, substituiert sein können.

2. Das Tropanderivat nach Anspruch 1, welches ist
(1 R,2R,3S)-2-(4-Chlorphenoxy-methyl)-3-(4-fluorphenyl)tropan;
(1R,2R,3S)-2-(4-Chlorphenoxy-methyl)-3-(3,4-dichlorphenyl)tropan;
(1R,2R,3S)-2-(4-Chlorphenoxy-methyl)-3-(4-methylphenyl)tropan; oder
(1R,2R,3S)-2-(Benzoyloxy-methyl)-3-(4-fluorphenyl)tropan;
oder ein pharmazeutisch verträgliches Additionssalz davon.

3. Verwendung eines Tropanderivats nach einem der Ansprüche 1-2 zur Herstellung eines Arzneimittels zur Behandlung einer Störung oder einer Krankheit des lebenden Tierkörpers, einschließlich dem Menschen, wobei die Störung oder die Krankheit auf die Inhibierung der Dopamin-Wiederaufnahme in dem zentralen Nervensystem anspricht.

4. Verwendung eines Tropanderivats nach einem der Ansprüche 1-2 zur Herstellung eines Arzneimittels zur Behandlung von Depressionen oder Parkinsonscher Krankheit.

5. Verwendung eines Tropanderivats nach einem der Ansprüche 1-2 zur Herstellung eines Medikaments zur Behandlung von Drogenabhängigkeit und/oder -missbrauch.

6. Verwendung eines Tropanderivats nach einem der Ansprüche 1-2 zur Herstellung eines Medikaments zur Behandlung von Kokain- und/oder Amphetaminabhängigkeit und/oder -missbrauch.

7. Verwendung nach einem der Ansprüche 3-6, worin das eingesetzte Tropanderivat ist
(1R,2R,3S)-2-(4-Chlorphenoxy-methyl)-3-(4-fluorphenyl)tropan;
(1R,2R,3S)-2-(4-Chlorphenoxy-methyl)-3-(3,4-dichlorphenyl)tropan;
(1R,2R,3S)-2-(4-Chlorphenoxy-methyl)-3-(4-methylphenyl)tropan; oder
(1R,2R,3S)-2-(Benzoyloxy-methyl)-3-(4-fluorphenyl)tropan;
oder ein pharmazeutisch verträgliches Additionssalz davon.

8. Pharmazeutische Zusammensetzung, umfassend eine wirksame Menge eines Tropanderivats nach einem der Ansprüche 1-2 oder ein pharmazeutisch verträgliches Additionssalz davon, zusammen mit wenigstens einem pharmazeutisch verträglichen Träger oder Verdünnungsmittel.

9. Verfahren zur Herstellung eines Tropanderivats nach einem der Ansprüche 1-2, wobei dieses Verfahren den Schritt des Reagierens einer Verbindung mit der folgenden Formel umfasst 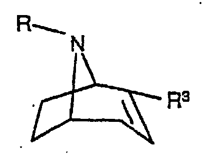 worin R und R³ die Bedeutungen besitzen wie in Anspruch 1 ausgeführt,
mit einer Verbindung mit der Formel R⁴-A,
worin R⁴ die Bedeutung besitzt wie in Anspruch 1 ausgeführt, und worin A eine beliebige Art einer reaktiven Funktionalität darstellt, die geeignet ist, ein Carbanion als ihr Gegenstück zu bilden, wie Li, MgX, worin X Halogen ist, und CuLi, in einer 1,4-Additionsreaktion vom Michael-Typ,
um ein erfindungsgemäßes Tropanderivat in einem Einschrittverfahren zu bilden.

## Revendications

1. Un dérivé 2,3-*trans*-isomérique du tropane de formule 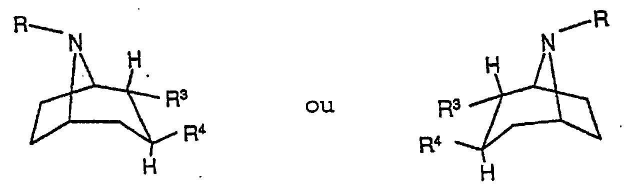 tout mélange dudit dérivé, ou un sel pharmaceutiquement acceptable dudit dérivé ;
dans laquelle
R est un hydrogène ou un alkyle en C₁₋₆ ;
R³ est le CH₂-O-phényle ou le CH₂-O-CO-phényle, lesquels groupes phényle peuvent être substitués une ou plusieurs fois par des substituants choisis dans le groupe consistant en un halogène, CF₃, CN, un alkoxy en C₁₋₆, un alkyle en C₁₋₆, NH₂ ou un nitro ; et
R⁴ est un phényle ou un benzyle, lesquels groupes phényle ou benzyle peuvent être substitués une ou plusieurs fois par des substituants choisis dans le groupe consistant en un halogène, CF₃, CN, un alkoxy en C₁₋₆, un alkyle en C₁₋₆, NH₂ ou un nitro.

2. Le dérivé du tropane selon la revendication 1, qui est :
le (1R,2R,3S)-2-(4-chlorophénoxy-méthyl)-3-(4-fluorophényl)tropane ;
le (1R,2R,3S)-2-(4-chlorophénoxy-méthyl)-3-(3,4-dichlorophényl)tropane ;
le (1R,2R,3S)-2-(4-chlorophénoxy-méthyl)-3-(4-méthylphényl) tropane ; ou
le (1R,2R,3S)-2-(benzoyloxy-méthyl)-3-(4-fluorophényl) tropane ;
ou un sel d'addition pharmaceutiquement acceptable dudit dérivé.

3. L'utilisation d'un dérivé du tropane selon l'une quelconque des revendications 1-2, pour la fabrication d'un médicament pour le traitement d'une affection ou d'une maladie d'un corps animal vivant, y compris un homme, laquelle affection ou maladie répond à l'inhibition de la recapture de la dopamine dans le système nerveux central.

4. L'utilisation d'un dérivé du tropane selon l'une quelconque des revendications 1-2, pour la fabrication d'un médicament pour le traitement de la dépression ou des syndromes parkinsoniens.

5. L'utilisation d'un dérivé du tropane selon l'une quelconque des revendications 1-2, pour la fabrication d'un médicament pour le traitement de la toxicomanie et/ou de l'usage abusif de drogues.

6. L'utilisation d'un dérivé du tropane selon l'une quelconque des revendications 1-2, pour la fabrication d'un médicament pour le traitement de la toxicomanie à la cocaïne et/ou aux amphétamines et/ou de l'usage abusif de cocaïne et/ou d'amphétamines.

7. L'utilisation selon l'une quelconque des revendications 3-6, dans laquelle le dérivé du tropane employé est :
le (1R,2R,3S)-2-(4-chlorophénoxy-méthyl)-3-(4-fluorophényl) tropane ;
le (1R,2R,3S)-2-(4-chlorophénoxy-méthyl)-3-(3,4-dichlorophényl) tropane ;
le (1R,2R,3S)-2-(4-chlorophénoxy-méthyl)-3-(4-méthylphényl) tropane ; ou
le (1R,2R,3S)-2-(benzoyloxy-méthyl)-3-(4-fluorophényl) tropane ;
ou un sel d'addition pharmaceutiquement acceptable dudit dérivé.

8. Une composition pharmaceutique comprenant une quantité efficace d'un dérivé du tropane selon l'une quelconque des revendications 1-2, ou un sel d'addition pharmaceutiquement acceptable dudit dérivé, avec au moins un véhicule ou diluant pharmaceutiquement acceptable.

9. Une méthode de préparation d'un dérivé du tropane selon l'une quelconque des revendications 1-2, laquelle méthode comporte une étape consistant à faire réagir un composé de formule : 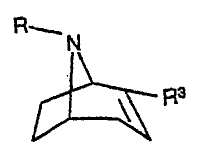 dans laquelle R et R³ ont les significations exposées dans la revendication 1,
avec un composé de formule R⁴-A,
dans laquelle R⁴ a la signification exposée dans la revendication 1 et dans laquelle A est tout type de fonctionnalité réactive convenant à la formation d'un carbanion lui servant de contrepartie, comme Li, MgX,
où X est un halogène, et CuLi, dans une réaction d'addition 1,4 de type Michael,
afin de former un dérivé du tropane selon l'invention par une procédure en une étape.
